# EUROPÄISCHE PATENTSCHRIFT

(11) **EP 1 263 974 B1**
(45) Veröffentlichungstag und Bekanntmachung des Hinweises auf die Patenterteilung: **11.08.2004**
(21) Anmeldenummer: 01915337.8
(22) Anmeldetag: 14.03.2001
(51) Int. Cl.: C12N 15/75, C12N 15/63, C12N 1/21, C12P 21/00

(54) **WIRTS-VEKTOR-SYSTEME ZUR PHOSPHATREGULIERTEN ÜBERPRODUKTION VON POLYPEPTIDEN IN BACILLUS**
HOST-VECTOR SYSTEMS FOR THE PHOSPHATE-REGULATED EXCESS PRODUCTION OF POLYPEPTIDES IN BACILLUS
SYSTEMES HOTES-VECTEURS POUR LA SURPRODUCTION REGULEE PAR PHOSPHATE DE POLYPEPTIDES DANS LES BACILLES

(30) Priorität: 14.03.2000 DE 10012283
(43) Veröffentlichungstag der Anmeldung: 11.12.2002
(73) Patentinhaber: Institut für Marine Biotechnologie E.V., 17489 Greifswald (DE)
(72) Erfinder: SCHWEDER, Thomas, 17489 Greifswald (DE); ANTELMANN, Haike, 18435 Stralsund (DE); HECKER, Michael, 17489 Greifswald (DE)
(74) Vertreter: Weiss, Wolfgang, Dipl.-Chem. Dr.
(86) Internationale Anmeldenummer: PCT/EP2001/002877
(87) Internationale Veröffentlichungsnummer: WO 2001/068883

(56) Entgegenhaltungen:
- US-A- 5 304 472
- STEPHANIE M. BIRKEY ET AL.: "Pho signal transduction network reveals direct transcriptional regulation of two-component system by another two-component regulator: Bacillus subtilis PhoP directly regulates production of ResD" MOLECULAR MICROBIOLOGY, Bd. 30, Nr. 5, 1998, Seiten 943-953, XP002171078 in der Anmeldung erwähnt

## Beschreibung

Die Erfindung betrifft durch Phosphathunger regulierbare *Bacillus*-Wirts-Vektor-Systeme zur Überproduktion rekombinanter Proteine.

Wenn anorganische Phosphatquellen im Medium verbraucht sind, produzieren viele Mikroorganismen alkalische Phosphatasen (APasen), um organisch gebundenes Phosphat zu mobilisieren. WO-A-94/12636 beschreibt ein durch Phosphatlimitation induzierbares Expressionssystem für das Gram-negative Bakterium *Escherichia coli.* Dieses System basiert auf einer mutierten Variante des Phosphat-Bindeproteins PstS sowie dem Promotor der Alkalischen Phosphatase von *E. coli, phoA.*

Das Gram-positive Bakterium *Bacillus subtilis* synthetisiert bei Phosphatlimitation mindestens zwei APasen, PhoA und PhoB (Hulett et al., 1994). Die Synthese beider APasen wird durch das Zwei-Komponenten-System PhoP-PhoR reguliert (Liu and Hulett, 1997; Liu et al., 1998a,b; Qui and Hulett, 1998a,b). Eine dritte APase, das Genprodukt von *phoD,* hat hauptsächlich Phosphodiesterase (APDase) - Aktivität (Eder et al., 1996).

Das Gram-positive Bakterium *B. subtilis* ist ein anerkannter industrieller Wirt. Dennoch fehlen für diesen Mikroorganismus geeignete, leicht regulierbare, starke Expressionssysteme. Es besteht daher ein großes Bedürfnis, solche Expressionssysteme bereitzustellen. Ein durch Phosphatlimitation induzierbares Expressionssystem in *B.subtilis* wurde bislang noch nicht beschrieben.

Die vorliegende Erfindung ist ein starkes, durch die Phosphatkonzentration im Medium reguliertes Expressionssystem, welches vorzugsweise auf einer Expressionskontrollsequenz eines durch Phosphatlimitation induzierbaren *Bacillus*-Gens basiert. Das System ermöglicht eine phosphatregulierte Überexpression von heterologen Genen in Gram-positiven Bakterien, insbesondere in Vertretern der Gattung *Bacillus*. Im Vergleich zu *E. coli* ist *B. subtilis* ein ausgewiesener Mikroorganismus der zur Sekretion von Fremdproteinen in das extrazelluläre Medium befähigt ist. Weiterhin ist *B. subtilis* ein GRAS-Organismus (GRAS = Generally Regarded As Safe), der keine Endotoxine bildet und damit besonders für die Überproduktion von pharmazeutischen sowie Nahrungsmittel-Enzymen geeignet ist.

Ein Gegenstand der vorliegenden Erfindung ist somit ein System zur phosphatregulierten Genexpression in Gram-positiven Organismen, insbesondere *Bacillus* umfassend
(a) eine Genexpressionseinheit enthaltend eine erste Expressionskontrollsequenz mit einem phosphatregulierbaren Promotor, einer ribosomalen Bindungsstelle und gegebenenfalls eine für ein Signalpeptid kodierenden Nukleinsäuresequenz,
(b) eine Regulationseinheit enthaltend ein PhoP-PhoR-Phosphatregulationssystem von Bacillus in operativer Verknüpfung mit einer zweiten Expressionskontrollsequenz.

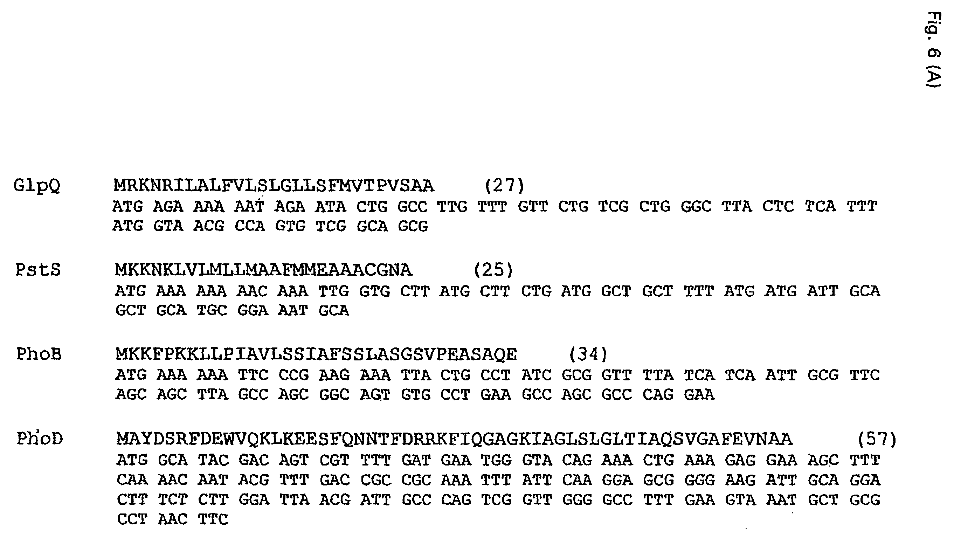

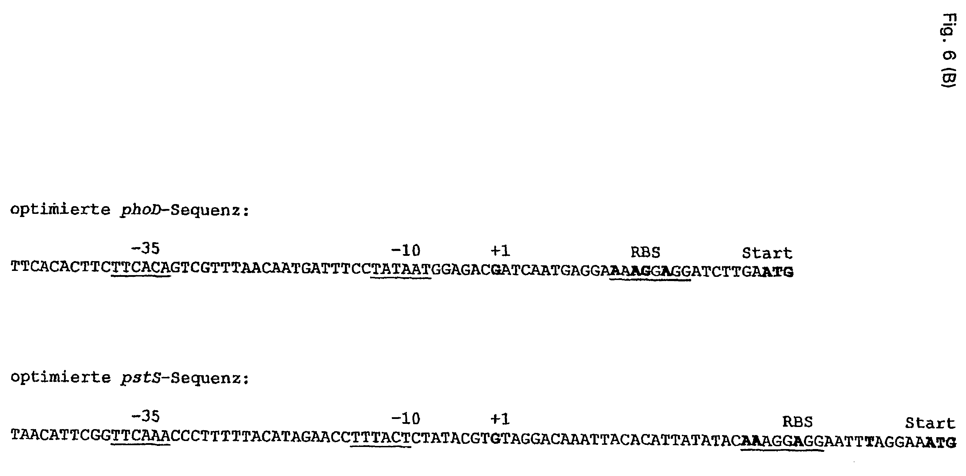

Der Promotor und gegebenenfalls die ribosomale Bindungsstelle der ersten Expressionssequenz stammen vorzugsweise von durch Phosphatlimiation regulierbaren Genen aus *Bacillus* z.B. von den Genen pstS, phoD, phoB oder glpQ von *B.subtilis.* Alternativ können auch funktionelle Varianten der *B.subtilis* Expressionskontrollsequenzen verwendet werden, die sich durch Substitution, Insertion oder/und Deletion einzelner Nukleotide oder Nukleotidabschnitte von den natürlichen Sequenzen unterscheiden. Bevorzugte Beispiele solcher funktionellen Varianten sind Expressionskontrollsequenzen umfassend die in Fig. 6B gezeigten Sequenzen, bei denen es sich um insbesondere hinsichtlich der ribosomalen Bindestelle optimierte Expressionskontrollsequenzen handelt.

Zusätzlich kann die erste Expressionskontrollsequenz stromabwärts der ribosomalen Bindungsstelle eine für ein Signalpeptid kodierende Nukleinsäuresequenz in operativer Verknüpfung mit Promotor und ribosomaler Bindungsstelle enthalten. Diese Signalpeptidsequenz ermöglicht die Expression von rekombinanten heterologen Proteinen, die von der Wirtszelle in das extrazelluläre Kulturmedium sekretiert werden.

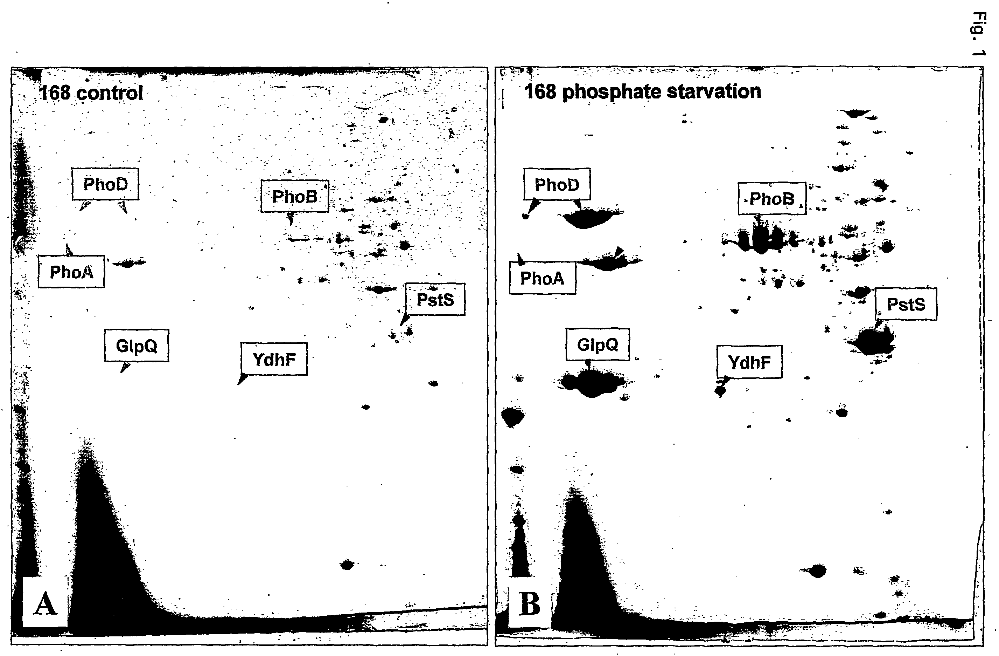

Das Signalpeptid und die dafür kodierende Nukleotidsequenz sind vorzugsweise von den Genen pstS, phoD, phoB oder glpQ von *B.subtilis* abgeleitet und umfassen vorzugsweise eine der in Fig. 6A gezeigten Sequenzen. Die in Figur 6A dargestellten Signalsequenzen von glpQ, pstS, phoB und phoD sind vor allem für eine Sekretion von Fremdgenen unter Phosphathungerbedingungen geeignet (s. auch Figur 1). Weiterhin können diese Signalsequenzen auch mit Fremdgenen fusioniert werden, die unter anderen Kultivierungsbedingungen in *Bacillus* überexprimiert werden, z.B. in der exponentiellen Wachstumsphase oder bei Glukoselimitation und dadurch eine Sekretion dieser Fremdproteine unter diesen Bedingungen ermöglichen. Das Startcodon der Signalsequenz von phoB kann dabei von TTG zu ATG verändert werden.

Die Genexpressionseinheit kann weiterhin eine Klonierungsstelle in operativer Verknüpfung mit der ersten Expressionskontrollsequenz enthalten, um eine Einklonierung eines gewünschten Zielgens, das für ein heterologes zu exprimierendes Polypeptid kodiert, zu ermöglichen. Vorzugsweise ist die Klonierungsstelle, die eine oder mehrere Restriktionsschnittstellen umfassen kann, so angeordnet, daß sie eine operative Verknüpfung eines einklonierten Zielgens mit der Expressionskontrollsequenz und insbesondere auch der Signalpeptidkodierenden Sequenz ermöglicht. In einer anderen Ausführungsform der Erfindung kann die Genexpressionseinheit bereits ein Strukturgen, das für ein heterologes zu exprimierendes Polypeptid kodiert, in operativer Verknüpfung mit der Expressionskontrollsequenz enthalten.

Die Regulationseinheit des erfindungsgemäßen Expressionssystems enthält ein PhoP-PhoR-Phosphatregulationssystem von *Bacillus,* insbesondere von *B.subtilis,* z.B. die Gene phoP (Accession Nr. X67676) und phoR (Accession Nr. M23549).

Das erfindungsgemäße Genexpressionssystem kann auf einem oder zwei Vektoren oder auch auch auf dem Chromosom einer Wirtszelle lokalisiert sein. Die Vektoren sind vorzugsweise Gram-positive prokaryontische Vektoren, d.h. Vektoren, die zur Propagierung in einer Gram-positiven prokaryontischen Wirtszelle, insbesondere in einer *B.subtilis* Wirtszelle fähig sind. Beispiele für derartige Vektoren sind Plasmidvektoren, Bakteriophagen, Cosmide etc. Besonders bevorzugt sind die Vektoren Derivate des Plasmids pAMβ1 aus *Enterococcus faecalis* (Bruand et al. EMBO J. 10 (1991); 2171-2177; Swinfield et al., Plasmid 26 (1991), 209-221). Weiterhin weisen die Vektoren vorzugsweise einen für die jeweilige Wirtszelle geeigneten Replikationsursprung sowie vorzugsweise ein Antibiotikumresistenzgen auf, um eine Selektion zu ermöglichen. Zur Optimierung der phosphatregulierten Genexpression ist die Klonierung der Regulatorgene phoP und phoR in operativer Verknüpfung mit einer zweiten Expressionskontrollsequenz auf demselben oder einem zweiten Vektor bevorzugt. Alternativ kann die Genexpressionseinheit auf einem extrachromosomalen Vektor lokalisiert oder in das Chromosom der Wirtszelle integriert sein, während die Regulationseinheit in das Chromosom der Wirtszelle, z.B. durch homologe Rekombination integriert ist.

Die Erfindung betrifft weiterhin eine Gram-positive Zelle, die in erfindungsgemäßes Expressionssystem enthält. Vorzugsweise ist die Zelle eine *B.subtilis* Zelle. Besonders bevorzugt ist die Wirtszelle eine sporulationsdefiziente *Bacillus* Zelle, insbesondere eine Bacillus spoOA-Mutante, wie sie bei Jensen et al. (J. Bacteriol, 175 (1993), 3749-3756) beschrieben ist, oder eine *Bacillus* spollA-Mutante.

Das erfindungsgemäße Expressionssystem und die erfindungsgemäße Zelle können in einem Verfahren zur gentechnischen Herstellung von Polypeptiden in Gram-positiven prokaryontischen Zellen verwendet werden.

Die Erfindung betrifft somit ein Verfahren zur gentechnischen Herstellung von Polypeptiden in einer prokaryontischen Zelle, welches dadurch gekennzeichnet ist, daß man ein phosphatregulierbares Expressionssystem wie zuvor beschrieben verwendet. Vorzugsweise umfaßt das Verfahren die Schritte
(i) Bereitstellen einer Zelle, die ein erfindungsgemäßes Expressionssystem enthält,
(ii) Kultivieren der Zelle aus (i) in einem geeigneten Medium und unter geeigneten Bedingungen, die zu einer Expression des gewünschten Polypeptids führen und
(iii) Isolieren des Polypeptids aus der Zelle oder vorzugsweise aus dem Medium.

Die Kultivierung der Zelle in Schritt (ii) des erfindungsgemäßen Verfahrens wird vorzugsweise auf solche Weise durchgeführt, daß bis zum Erreichen einer vorbestimmten Zelldichte die Expression des für das gewünschte Polypeptid kodierenden Gens weitgehend reprimiert ist, d.h. unter Bedingungen, bei denen eine Phosphatkonzentration im Medium vorliegt, bei der die erste Expressionskontrollsequenz reprimiert ist. Die Phosphatkonzentration beträgt vorzugsweise 10 bis 100 mM, besonders bevorzugt 10 bis 30 mM. Nach Erreichen einer bestimmten Zelldichte wird durch Phosphatlimitation, z.B. durch Medienwechsel oder Abfangen des im Medium vorhandenen Phosphats, z.B. durch Komplexbildung, die Expression des gewünschten Polypeptids induziert. Vorzugsweise wird die Phosphatlimitation, z.B. in einer Fed-Batch-Fermentation, durch Fütterung einer phosphatfreien oder -armen Nährlösung während der Fermentation oder/und durch Vorlage einer begrenzten Phosphatkonzentration im Kulturmedium bestimmt.

Die Erfindung wird weiterhin durch folgende Figuren und Beispiele erläutert.
Es zeigen:
Figur 1: Eine zweidimensionale Auftrennung der extrazellulären Proteine von *Bacillus subtilis* vor (A) und (B) 2 h nach Eintritt der stationären Phase durch Phosphatlimitation
Figur 2: Die APase-Aktivität von *B. subtilis* im Medium im Verlauf einer phosphatlimitierten Batch-Kultur.
Figur 3: Eine Northernanalyse der *glpQ* mRNA vor und 0.5, 1, 1.5, 2 sowie 2.5 h nach Eintritt der stationären Phase ausgelöst durch Phosphatlimitation.
Figur 4 (A): Die Identifizierung des Promotors von *glpQ* durch Primerextension. (B) Die Sequenz der abgeleitete *glpQ*-Promotorregion.
Figur 5: Die Stärke der Promotoren und der Translationssignale der phosphatregulierten Proteine PstS, PhoD, PhoB und GlpQ wurde mit Hilfe translationaler *lacZ*-Fusionen in *B. subtilis* analysiert.
Figur 6: (A) Mit Hilfe von Computerprogrammen wurden die potentiellen Signalpeptid-Sequenzen von GlpQ, PstS, PhoD und PhoB ermittelt. (B) Optimierte *phoD*- und *pstS*-Sequenzen.
Figur 7: Einen Vergleich der Stärke der Expression des Modellproteins ß-Galactosidase (LacZ) durch die optimierte *pstS*-Sequenz (vergl. Figur 6B) mit einer Wildtyp-*lacZ* Fusion.
Figur 8: Die Expression der ß-Galactosidase mit dem erfindungsgemäßen Expressionssystem während einer zweistufigen Fed-Batch Fermentation.

### Beispiele

### 1. Phosphatregulierte Genexpression in B.subtilis

Durch zweidimensionale Proteingelelektrophorese und anschließende Identifizierung der Proteinspots mittels MALDI-TOF Massenspektrometrie konnte die Synthese von Proteinen, die durch Phosphathunger reguliert werden, in *B. subtilis* genauer analysiert werden (Figur 1).

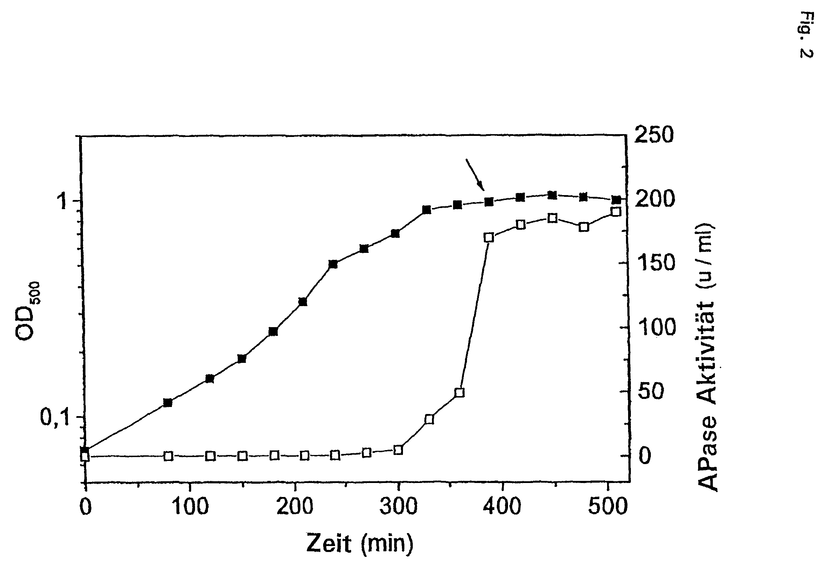

Durch diese Technik konnte eine starke Expression von verschiedenen Proteinen in der stationären Phase, ausgelöst durch Phosphatlimitation, vor allem im extrazellulären Medium beobachtet werden. Dies schließt die APasen PhoB, die APase/APDase PhoD, eine Glyrerophosphoryldiesterase (GlpQ) sowie das Phosphatbindeprotein PstS ein. GlpQ wurde als neues Mitglied des PhoPR-Regulons identifiziert. Alle phosphatregulierten Proteine werden in der exponentiellen Wachstumsphase, wenn Phosphat ausreichend im Medium vorhanden ist, nicht oder nur in sehr geringen Mengen synthetisiert. 30 Minuten nach Eintritt der stationären Phase, ausgelöst durch den Verbrauch von Phosphat im Medium, wird die Synthese dieser Proteine drastisch verstärkt. Die APase-Aktivität im Medium wird um den Faktor 200 erhöht (Figur 2).

Die Menge der durch Phosphathunger regulierten Proteine im extrazellulären Medium entspricht 1 h nach Eintritt der stationären Phase ca. 60-70 % des extrazellulären Gesamtproteins (Figur 1). Dabei sind vor allem die Proteine PhoB, PhoD, GlpQ und PstS hervorzuheben. Das Phosphatbindeprotein PstS ist unter diesen Bedingungen außerdem in großen Mengen im Cytoplasma zu finden (Eymann et al. 1996). Für *pstS* wurde eine 5000-fache Induktion des Promotors nach Phosphatlimitation beschrieben (Qi et al. 1998). Der Promotor wurde durch Primerextension identifiziert.

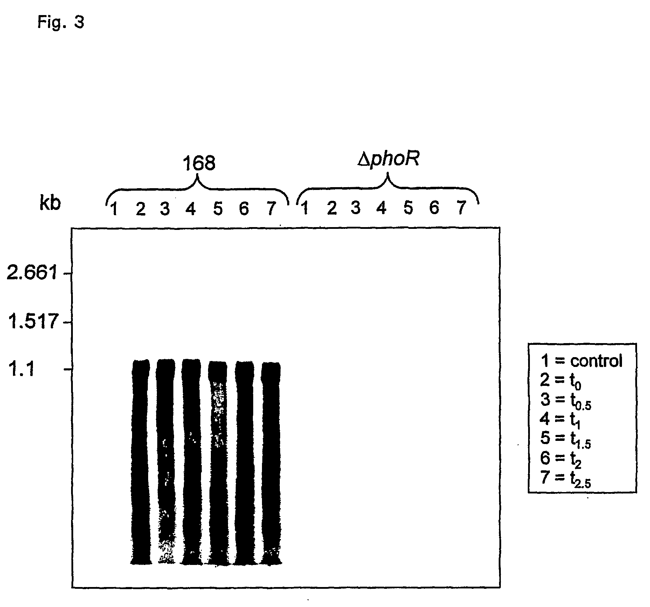

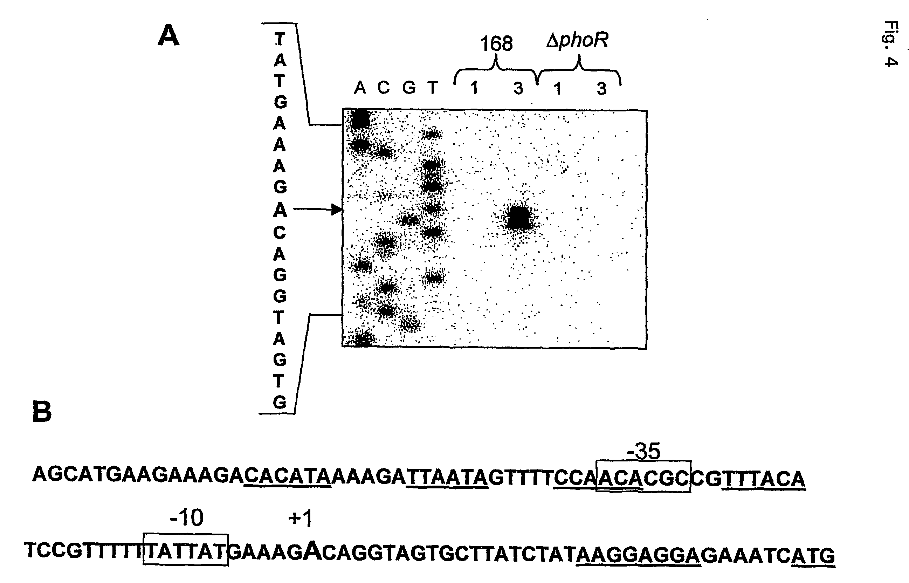

Außerdem konnte eine starke Expression von *glpQ* mittels Northernanalysen beobachtet werden (Figur 3). GlpQ wird hauptsächlich monocistronisch nach Phosphatlimitation transkribiert. Der Promotor wurde durch Primerextension identifiziert (Figur 4).

### 2. Bereitstellung von Expressionskontrollsequenzen für die phosphatregulierte Expression heterologer Proteine

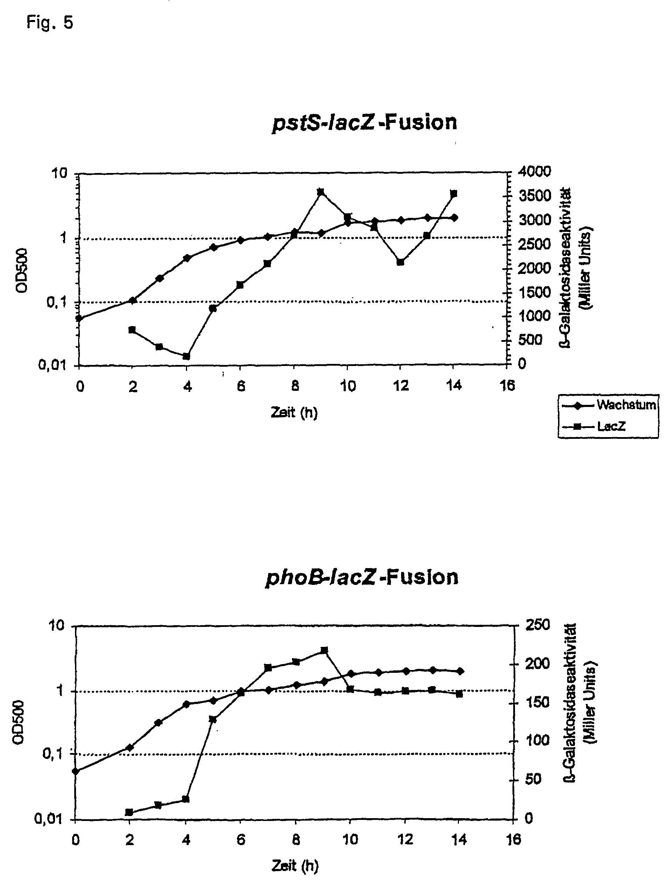

Die Stärke der Promotoren und der Translationssignale der phosphatregulierten Proteine PstS, PhoD, PhoB und GlpQ wurde mit Hilfe translationaler *lacZ*-Fusionen in *B. subtilis* analysiert (Figur 5).

Mit Hilfe von Computerprogrammen wurden die potentiellen Signalpeptid-Sequenzen dieser Proteine ermittelt (Figur 6A). Weiterhin konnten optimierte Sequenzen der Transkriptions- und Translationssignale bestimmt werden (Figur 6B).

### 3. Bereitstellung von Wirtszellen für die phosphatregulierte Genexpression

Als Ausgangsquelle kann ein sporulationsdefizienter spoOA *Bacillus*-Stamm (Jensen et al. (1993), supra) oder ein sporulationsdefizienter spollA Stamm verwendet werden. Eine Kopie der Gene phoP und phoR kann nach der Amplifikation mit Hilfe der PCR-Technik in das Amylasegen von *Bacillus subtilis* auf einem *Escherichia coli* Plasmid integriert, das nur zur Replikation in *E. coli* aber nicht in *Bacillus* befähigt ist, integriert werden. Nach Linearisierung und Transformation dieses Plasmids in *Bacillus,* kann die phoP-phoR-Sequenz durch die flankierenden Amylasegensequenzen über zwei Crossingover in das chromosomale Amylasegen von *B.subtilis* integriert werden.

### 4. Phosphatregulierte Expression heterologer Proteine

Die Stärke der Expression des Modellproteins β-Galaktosidase (LacZ) mit den optimierten *phoD*- und *pstS*-Sequenzen wurde mit den Wildtyp *lacZ*-Fusionen verglichen.

Zur Konstruktion der *lacZ*-Fusionen wurden die jeweiligen Promotorsequenzen mit Hilfe der unten angegebenen Primer amplifiziert, mit *EcoRI* und *BamHI* geschnitten und in den Translationsfusionvektor pAC7 eingebaut (Ref.: Weinrauch Y, Msadek T, Kunst F, Dubnau D 1991. Sequence and properties of *comQ,* a new competence regulatory gene of *Bacillus subtilis*. J Bacteriol 173(18):5685-93). Nach Linearisierung der Vektoren mit *Scal* und einer Transformation in *Bacillus* wurden die entsprechenden *lacZ*-Fusionen in den *amyE*-Ort des *Bacillus*-Chromosoms einrekombiniert.

Die folgenden Primer wurden für die Konstruktion der lacZ-Fusionen mit den Phosphathunger-induzierbaren Promotoren verwendet.

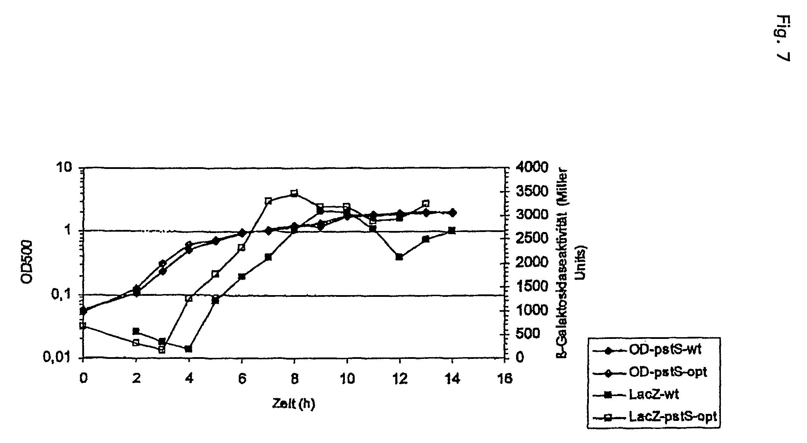

Die verschiedenen *lacZ*-Fusionen wurden in Phosphat-limitierten BLM-Medium mit 0,1 mM Phosphat getestet. Die höchste *β*-Galactosidaseaktivität zeigte die Fusion mit dem *pstS*-Promotor und den optimierten Translationssignalen (Figur 5, 7). Die Bestimmung der β-Galaktosidaseaktivität erfolgte nach Kenney and Moran (Kenney, T.J. and Moran, C.P., Jr (1987) Organization and regulation of an operon that encodes a sporulation-essential sigma factor in *Bacillus subtilis J. Bacteriol.* 169, 3329-3339). Die Aktivität wurde als Miller Units per optischer Dichte dargestellt.

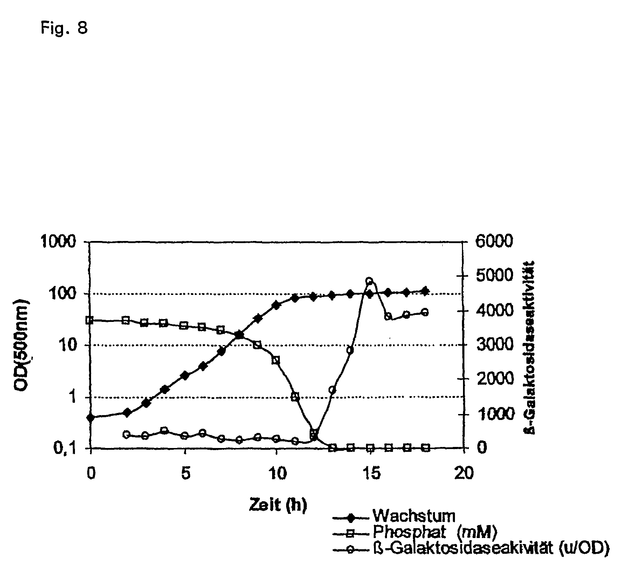

Die Expression der optimierten *pst-lacZ*-Fusion wurde in einer Fed-Batch Fermentation getestet. (Figur 8). Der Fermentationsprozeß, mit dem in dieser Erfindung beschriebenen phosphatregulierten Expressionssystemen wird zweistufig durchgeführt. Die erste Phase, die sogenannte Wachtumsphase, wird als Fed-Batch Fermentation durchgeführt. In dieser Phase ist im Kulturmedium oder/und in der Fütterungslösung ausreichend Phosphat enthalten. Das Grundmedium enthält vorzugsweise die folgende Zusammensetzung:

| | Endkonzentration | g/l |
|---|---|---|
| Glukose | | 5 |
| Tris | 50 mM | 6,057 |
| (NH₄)₂SO₄ | 15 mM | 2 |
| MgSO₄ x 7 H₂O | 8 mM | 2 |
| KCI | 27 mM | 2 |
| Natriumcitrat x 2 H₂O | 7 mM | 2,1 |
| Glutaminsäure | 9 mM | 0,825 |

Der pH-Wert der Salzlösung wird auf 7.5 eingestellt (mit HCI). Nach dem Autoklavieren werden folgende Salze dazugeben:

| | Stammlösung | g/ml | Endkonz. | ml/100ml |
|---|---|---|---|---|
| CaCl₂ | 1 M | 7,35 g/50ml | 2mM | 0,2 |
| FeSO₄ | 0,5 mM | 6,95g/50ml | 1 µM | 0,2 |
| MnSO₄ | 25 mM | 0,275g/50ml | 19 µM | 0,04 |
| KH₂PO₄ | 0,2 M | 1,35g/50ml | 0,4 mM | 0,2 |

Nach dem Verbrauch der zu Beginn der Fermentation vorgelegten Glukose (5 g/l), identifiziert durch den Anstieg des pO₂-Levels wurde kontinuierlich Glukoselösung zugefüttert. Die Fütterungslösung enthielt (in g/kg Lösung): Glukose 200, Tris 6, (NH₄)₂SO₄ 2.0, Glutaminsäure 0.9, KCI 0.5, Na-Citrat x 2H₂O 1.0, und Spurenelementelösung 10 mL kg⁻¹. Zusätzlich wurde 2.75 m/l Lösung MgSO₄ (1 M) zweimal während der Fed-Batch Kultivierung separat zugegeben, um ein Ausfallen von Salz in der Fütterungslösung zu verhindern.

In dem in Figur 8 gezeigten Fermentationsprozeß wurde 20 mM Phosphat in das Medium gegeben. Dadurch wurde eine optische Dichte (OD₅₀₀ₙₘ) von etwa 100 erreicht bis das Phosphat verbraucht war, und der pstS-Promotor induziert wurde. Die maximale β-Galactosidaseaktivität war in der Fermentation nach etwa 5 h nach Phosphatlimitation zu beobachten (Fig. 8).

In der zweiten Phase, der Produktionsphase, ist entweder im Kulturmedium das Phosphat verbraucht oder es wird nun eine Fütterungslösung zugegeben, die kein Phosphat mehr enthält. Durch den damit ausgelösten Phosphathunger wird eine Induktion des Expressionssystems über das Zweikomponentensystem PhoP-PhoR erreicht. In bestimmten Zeitabständen ist eine limitierte Zugabe von Phosphat, insbesondere von organischen Phosphatverbindungen, möglich, um die durch Phosphathunger ausgelöste Überproduktion der entsprechenden Polypeptide zu optimieren.

### Literatur

**Birkey S.M., W. Liu, X. Zhang, M.F. Duggan and F.M. Hulett.** 1998. Pho signal transduction network reveals direct transcriptional regulation of one two-component system by another two-component regulator: *Bacillus subtilis* PhoP directly regulates production of ResD. Mol Microbiol. **30:** 943-953.
**Eder S, W. Liu, F.M. Hulett.** 1999. Mutational analysis of the *phoD* promoter in *Bacillus subtilis:* implications for PhoP binding and promoter activation of Pho regulon promoters. J. Bacteriol. **181:** 2017-2025.
**Eder S., L. Shi, K. Jensen, K. Yamane, F.M. Hulett.** 1996. A *Bacillus subtilis* secreted phosphodiesterase/alkaline phosphatase is the product of a Pho regulon gene, *phoD.* Microbiology **142:** 2041-2047.
**Eymann C., H. Mach, C.R. Harwood and M. Hecker.** 1996. Phosphatestarvation-inducible proteins in *Bacillus subtilis*: a two-dimensional gel electrophoresis study. Microbiology **142:** 3163-3170.
**Hulett, F. M., J. Lee, L. Shi, G. Sun, R. Chesnut, E. Sharkova, M. F. Duggan, and N. Kapp.** 1994. Sequential action of two-component genetic switches regulates the Pho regulon in *Bacillus subtilis*. J. Bacteriol. **176:** 1348-1358.
**Liu W. and F.M. Hulett.** 1997. *Bacillus subtilis* PhoP binds to the *phoB* tandem promoter exclusively within the phosphate starvation-inducible promoter. J. Bacteriol. 179: 6302-10.
**Liu W. and F.M. Hulett.** 1998. Comparison of PhoP binding to the *tuaA* promoter with PhoP binding to other Pho-regulon promoters establishes a *Bacillus subtilis* Pho core binding site. Microbiology **144:** 1443-1450.
**Liu W., Y. Qi and F.M. Hulett.** 1998. Sites internal to the coding regions of *phoA* and *pstS* bind PhoP and are required for full promoter activity. Mol Microbiol. 28: 119-130.
**Nilsson R.P., L. Beijer, and B. Rutberg.** 1994. The *glpT* and *glpQ* genes of the glycerol regulon in *Bacillus subtilis*. Microbiology 140: 723-730.
**Qi Y., and F.M. Hulett.** 1998. PhoP-P and RNA polymerase holoenzyme are sufficient for transcription of Pho. regulon promoters in *Bacillus subtilis*: PhoP-P activator sites within the coding region stimulate transcription in vitro. Mol Microbiol. **28:** 1187-1197.
**Qi Y., and F.M. Hulett.** 1998. Role of Pho-P in transcriptional regulation of genes involved in cell wall anionic polymer biosynthesis in *Bacillus subtilis*. J. Bacteriol. 180: 4007-4010.
**Seki T., H. Yoshikawa, H. Takahashi, and H. Saito.** 1987. Cloning and nucleotide sequence of *phoP,* the regulatory gene for alkaline phosphatase and phosphodiesterase in *Bacillus subtilis*. J Bacteriol. **169:** 2913-2916.
**Shi L., and F.M. Hulett.** 1999. The cytoplasmic kinase domain of PhoR is sufficient for the low phosphate-inducible expression of pho regulon genes in *Bacillus subtilis*. Mol Microbiol. **31:** 211-222.

## Patentansprüche

1. System zur phosphatregulierten Genexpression von Gram-positiven Organismen umfassend
(a) eine Genexpressionseinheit enthaltend eine erste Expressionskontrollsequenz mit einem phosphatregulierbaren Promotor und einer ribosomalen Bindungsstelle,
(b) eine Regulationseinheit enthaltend ein PhoP-PhoR-Phosphatregulationssystem von *Bacillus* in operativer Verknüpfung mit einer zweiten Expressionskontrollsequenz.

2. System nach Anspruch 1,
**dadurch gekennzeichnet,**
**daß** Promotor und gegebenenfalls die ribosomale Bindungsstelle der ersten Expressionskontrollsequenz von den Genen pstS, phoD, phoB oder glpQ von *B.subtilis* stammen oder funktionelle Varianten davon sind, die sich durch Substitution, Insertion und/oder Deletion einzelner Nukleotide oder Nukleotidabschnitte von den natürlichen Sequenzen unterscheiden.

3. System nach Anspruch 2,
**dadurch gekennzeichnet,**
**daß** die erste Expressionskontrolisequenz eine der in Fig. 6B gezeigten Sequenzen umfaßt.

4. System nach einem der Ansprüche 1 bis 3,
**dadurch gekennzeichnet,**
**daß** die erste Expressionskontrollsequenz weiterhin eine für ein Signalpeptid zur Sekretion in das extrazelluläre Medium kodierende Nukleinsäuresequenz in operativer Verknüpfung mit Promotor und ribosomaler Bindungsstelle enthält.

5. System nach Anspruch 4,
**dadurch gekennzeichnet,**
**daß** das Signalpeptid von den Genen pstS, phoD, phoB oder glpQ von *B.subtilis* abgeleitet ist.

6. System nach Anspruch 5,
**dadurch gekennzeichnet,**
**daß** das Signalpeptid eine der Fig. 6A gezeigten Sequenzen umfaßt.

7. System nach einem der vorhergehenden Ansprüche,
**dadurch gekennzeichnet,**
**daß** die Genexpressionseinheit (a) weiterhin eine Klonierungsstelle in operativer Verknüpfung mit der ersten Expressionskontrollsequenz enthält.

8. System nach einem der vorhergehenden Ansprüche,
**dadurch gekennzeichnet,**
**daß** die Genexpressionseinheit (a) weiterhin ein Strukturgen in operativer Verknüpfung mit der ersten Expressionskontrollsequenz enthält.

9. Gram-positive Zelle enthaltend ein Expressionssystem nach einem der Ansprüche 1 bis 8.

10. Zelle nach Anspruch 9,
**dadurch gekennzeichnet,**
**daß** sie eine *B.subtilis* Zelle ist.

11. Verfahren zur gentechnischen Herstellung von Polypeptiden in einer Gram-positiven prokaryontischen Zelle,
**dadurch gekennzeichnet,**
**daß** man ein phosphat-regulierbares Expressionssystem nach einem der Ansprüche 1 bis 9 verwendet.

12. Verfahren nach Anspruch 11,
**dadurch gekennzeichnet,**
**daß** das Expressionssystem durch die Limitation einer anorganischen Phosphatquelle im Kulturmedium induziert wird.

13. Verfahren nach Anspruch 12,
**dadurch gekennzeichnet,**
**daß** die Limitation der anorganischen Phosphatquelle in einer Fed-Batch-Fermentation durch Fütterung einer phosphatfreien Nährlösung oder/und durch Vorlage einer begrenzten Phosphatkonzentration im Kulturmedium bestimmt wird.

## Claims

1. System for phosphate-regulated gene expression by gram-positive organisms comprising
(a) a gene expression unit containing a first expression control sequence with a phosphate-regulatable promoter and a ribosomal binding site,
(b) a regulation unit containing a PhoP-PhoR phosphate regulation system of *Bacillus* in operative linkage with a second expression control sequence.

2. System as claimed in claim 1,
**characterized in that**
the promoter and optionally the ribosomal binding site of the first expression control sequence are derived from the genes pstS, phoD, phoB or glpQ of *B.subtilis* or are functional variants thereof that differ from the natural sequences by substitution, insertion and/or deletion of individual nucleotides or nucleotide sections.

3. System as claimed in claim 2,
**characterized in that** the first expression control sequence comprises one of the sequences shown in fig. 6B.

4. System as claimed in one of the claims 1 to 3,
**characterized in that**
the first expression control sequence additionally contains a nucleic acid sequence coding for a signal peptide for secretion into the extracellular medium in operative linkage with a promoter and ribosomal binding site.

5. System as claimed in claim 4,
**characterized in that**
the signal peptide is derived from the genes pstS, phoD, phoB or glpQ of *B.subtilis.*

6. System as claimed in claim 5,
**characterized in that**
the signal peptide comprises one of the sequences shown in fig. 6A.

7. System as claimed in one of the previous claims,
**characterized in that**
the gene expression unit (a) additionally contains a cloning site in operative linkage with the first expression control sequence.

8. System as claimed in one of the previous claims,
**characterized in that**
the gene expression unit (a) additionally contains a structural gene in operative linkage with the first expression control sequence.

9. Gram-positive cell containing an expression system as claimed in one of the claims 1 to 8.

10. Cell as claimed in claim 9,
**characterized in that**
it is a *B.subtilis* cell.

11. Process for the production of polypeptides in a gram-positive prokaryotic cell by genetic engineering,
**characterized in that**
a phosphate-regulatable expression system as claimed in one of the claims 1 to 9 is used.

12. Method as claimed in claim 11,
**characterized in that**
the expression system is induced by limitation of an inorganic phosphate source in the culture medium.

13. Method as claimed in claim 12,
**characterized in that**
the limitation of the inorganic phosphate source is achieved in a fed-batch fermentation by feeding a phosphate-free nutrient solution or/and by providing a limited phosphate concentration in the culture medium.

## Revendications

1. Système pour l'expression génétique régulée par phosphate d'organismes Gram-positif comprenant :
(a) une unité d'expression génétique comprenant une première séquence de contrôle de l'expression avec un promoteur régulable par phosphate et un site de liaison ribosomal,
(b) une unité de régulation comprenant un système de régulation par phosphate PhoP-PhoR de *Bacillus* en liaison opérationnelle avec une deuxième séquence de contrôle de l'expression.

2. Système selon la revendication 1, **caractérisé en ce que** le promoteur et le cas échéant le site de liaison ribosomal de la première séquence de contrôle de l'expression proviennent des gènes pstS, phoD, phoB ou glpQ de *B. subtilis* ou sont des variantes fonctionnelles de ceux-ci, qui se distinguent des séquences naturelles par substitution, insertion et/ou délétion de nucléotides isolés ou de segments de nucléotides.

3. Système selon la revendication 2, **caractérisé en ce que** la première séquence de contrôle de l'expression comprend l'une des séquences présentées à la Figure 6B.

4. Système selon l'une des revendications 1 à 3, **caractérisé en ce que** la première séquence de contrôle de l'expression comprend en outre une séquence d'acides nucléiques codante pour un peptide signal pour la sécrétion dans le milieu extracellulaire, en liaison opérationnelle avec le promoteur et le site de liaison ribosomal

5. Système selon la revendication 4, **caractérisé en ce que** le peptide signal est dérivé des gènes pstS, phoD, phoB ou glpQ de *B. subtilis.*

6. Système selon la revendication 5, **caractérisé en ce que** le peptide signal comprend l'une des séquences présentées à la Figure 6A.

7. Système selon l'une des revendications précédentes, **caractérisé en ce que** l'unité d'expression génétique (a) comprend en outre un site de clonage en liaison opérationnelle avec la première séquence de contrôle de l'expression.

8. Système selon l'une des revendications précédentes, **caractérisé en ce que** l'unité d'expression génétique (a) comprend en outre un gène de structure en liaison opérationnelle avec la première séquence de contrôle de l'expression.

9. Cellule Gram-positif comprenant un système d'expression selon l'une des revendications 1 à 8.

10. Cellule selon la revendication 9, **caractérisée en ce qu'**elle est une cellule de *B. subtilis.*

11. Procédé pour la production par génie génétique de polypeptides dans une cellule Gram-positif procaryote, **caractérisé en ce qu'**on utilise un système d'expression régulable par phosphate selon l'une des revendications 1 à 9.

12. Procédé selon la revendication 11, **caractérisé en ce que** le système d'expression est induit par la limitation d'une source de phosphate inorganique dans le milieu de culture.

13. Procédé selon la revendication 12, **caractérisé en ce que** la limitation de la source de phosphate inorganique dans une fermentation à écoulement discontinu est déterminée par l'apport d'une solution nutritive sans phosphate ou/et par exposition à une concentration en phosphate limitée dans le milieu de culture.


### SEQUENZPROTOKOLL

<110> Institut für Marine Biotechnologie e.V.
<120> Wirts-Vektor-Systeme zur phosphatregulierten Überproduktion von Polypeptiden in Bacillus
<130> 22088P WO_Schweder
<140> PCT/EP 01/02877
   <141> 2001-03-14
<150> DE 100 12 283.3
   <151> 2000-03-14
<160> 21
<170> PatentIn Ver. 2.1
<210> 1
   <211> 109
   <212> DNA
   <213> Bacillus subtilis
<220>
   <221> -35_signal
   <222> (41)..(47)
<220>
   <221> -10_signal
   <222> (65)..(70)
<220>
   <221> RBS
   <222> (95)..(102)
<400> 1
   agcatgaaga aagacacata aaagattaat agttttccaa cacgccgttt acatccgttt 60
   tttattatga aagacaggta gtgcttatct ataaggagga gaaatcatg             109
<210> 2
   <211> 81
   <212> DNA
   <213> Bacillus subtilis
<220>
   <221> sig_peptide
   <222> (1)..(81)
<220>
   <221> CDS
   <222> (1)..(81)
<400> 2
   atg aga aaa aat aga ata ctg gcc ttg ttt gtt ctg tcg ctg ggc tta    48
   Met Arg Lys Asn Arg Ile Leu Ala Leu Phe Val Leu Ser Leu Gly Leu
    1               5                  10                  15
   ctc tca ttt atg gta acg cca gtg tcg gca gcg                         81
   Leu Ser Phe Met Val Thr Pro Val Ser Ala Ala
               20                  25
<210> 3
   <211> 27
   <212> PRT
   <213> Bacillus subtilis
<400> 3
   Met Arg Lys Asn Arg Ile Leu Ala Leu Phe Val Leu Ser Leu Gly Leu
    1               5                  10                  15
   Leu Ser Phe Met Val Thr Pro Val Ser Ala Ala
               20                  25
<210> 4
   <211> 75
   <212> DNA
   <213> Bacillus subtilis
<220>
   <221> sig_peptide
   <222> (1)..(75)
<220>
   <221> CDS
   <222> (1)..(75)
<400> 4
   atg aaa aaa aac aaa ttg gtg ctt atg ctt ctg atg gct gct ttt atg    48
   Met Lys Lys Asn Lys Leu Val Leu Met Leu Leu Met Ala Ala Phe Met
    1               5                  10                  15
   atg att gca gct gca tgc gga aat gca                                 75
   Met Ile Ala Ala Ala Cys Gly Asn Ala
               20                  25
<210> 5
   <211> 25
   <212> PRT
   <213> Bacillus subtilis
<400> 5
   Met Lys Lys Asn Lys Leu Val Leu Met Leu Leu Met Ala Ala Phe Met
   1               5                   10                  15
   Met Ile Ala Ala Ala Cys Gly Asn Ala
               20                  25
<210> 6
   <211> 102
   <212> DNA
   <213> Bacillus subtilis
<220>
   <221> sig_peptide
   <222> (1)..(102)
<220>
   <221> CDS
   <222> (1)..(102)
<400> 6
   atg aaa aaa ttc ccg aag aaa tta ctg cct atc gcg gtt tta tca tca     48
   Met Lys Lys Phe Pro Lys Lys Leu Leu Pro Ile Ala Val Leu Ser Ser
   1               5                   10                  15
   att gcg ttc agc agc tta gcc agc ggc agt gtg cct gaa gcc agc gcc     96
   Ile Ala Phe Ser Ser Leu Ala Ser Gly Ser Val Pro Glu Ala Ser Ala
               20                  25                  30
   cag gaa                                                             102
   Gln Glu
<210> 7
   <211> 34
   <212> PRT
   <213> Bacillus subtilis
<400> 7
   Met Lys Lys Phe Pro Lys Lys Leu Leu Pro Ile Ala Val Leu Ser Ser
   1               5                   10                  15
   Ile Ala Phe Ser Ser Leu Ala Ser Gly Ser Val Pro Glu Ala Ser Ala
               20                  25                  30
   Gln Glu
<210> 8
   <211> 78
   <212> DNA
   <213> Bacillus subtilis
<220>
   <221> -35_signal
   <222> (11)..(16)
<220>
   <221> -10_signal
   <222> (37)..(42)
<220>
   <221> RBS
   <222> (61)..(68)
<220>
   <221> mutation
   <222> (61)
<220>
   <221> mutation
   <222> (63)..(64)
<220>
   <221> mutation
   <222> (66)
<400> 8
   ttcacacttc ttcacagtcg tttaacaatg atttcctata atggagacga tcaatgagga 60
   aaaggaggat cttgaatg                                               78
<210> 9
   <211> 94
   <212> DNA
   <213> Bacillus subtilis
<220>
   <221> -35_signal
   <222> (11)..(16)
<220>
   <221> -10_signal
   <222> (36)..(41)
<220>
   <221> RBS
   <222> (75)..(82)
<220>
   <221> mutation
   <222> (75)..(76)
<220>
   <221> mutation
   <222> (80)
<220>
   <221> mutation
   <222> (87)
<400> 9
   taacattcgg ttcaaaccct ttttacatag aacctttact ctatacgtgt aggacaaatt 60
   acacattata tacaaaggag gaatttagga aatg                              94
<210> 10
   <211> 180
   <212> DNA
   <213> Bacillus subtilis
<220>
   <221> CDS
   <222> (1)..(171)
   <223> Sig.-peptide
<400> 10
   atg gca tac gac agt cgt ttt gat gaa tgg gta cag aaa ctg aaa gag   48
   Met Ala Tyr Asp Ser Arg Phe Asp Glu Trp Val Gln Lys Leu Lys Glu
    1               5                  10                  15
   gaa agc ttt caa aac aat acg ttt gac cgc cgc aaa ttt att caa gga   96
   Glu Ser Phe Gln Asn Asn Thr Phe Asp Arg Arg Lys Phe Ile Gln Gly
               20                  25                  30
   gcg ggg aag att gca gga ctt tct ctt gga tta acg att gcc cag tcg   144
   Ala Gly Lys Ile Ala Gly Leu Ser Leu Gly Leu Thr Ile Ala Gln Ser
           35                  40                  45
   gtt ggg gcc ttt gaa gta aat gct gcg cctaacttc                     180
   Val Gly Ala Phe Glu Val Asn Ala Ala
       50                  55
<210> 11
   <211> 57
   <212> PRT
   <213> Bacillus subtilis
<400> 11
   Met Ala Tyr Asp Ser Arg Phe Asp Glu Trp Val Gln Lys Leu Lys Glu
     1               5                  10                  15
   Glu Ser Phe Gln Asn Asn Thr Phe Asp Arg Arg Lys Phe Ile Gln Gly
                20                  25                  30
   Ala Gly Lys Ile Ala Gly Leu Ser Leu Gly Leu Thr Ile Ala Gln Ser
            35                  40                  45
   Val Gly Ala Phe Glu Val Asn Ala Ala
        50                  55
<210> 12
   <211> 29
   <212> DNA
   <213> Bacillus subtilis
<220>
   <223> phoB-forward-primer
<400> 12
   ggggaattca gatttcagtc cgcctatcc                                    29
<210> 13
   <211> 27
   <212> DNA
   <213> Bacillus subtilis
<220>
   <223> phoB-reverse-primer
<400> 13
   gggggatcca ttggataacc tcctaaa                                      27
<210> 14
   <211> 29
   <212> DNA
   <213> Bacillus subtilis
<220>
   <223> phoD-forward-primer
<400> 14
   ggggaattcg agtcattctg cgaaatggc                                    29
<210> 15
   <211> 29
   <212> DNA
   <213> Bacillus subtilis
<220>
   <223> phoD-mut-forward-primer
<400> 15
   ggggaattcg agtcattctg cgaaatggc 29
<210> 16
   <211> 35
   <212> DNA
   <213> Bacillus subtilis
<220>
   <223> phoD-mut-reverse-primer
<400> 16
   gggggatcca ttcaagatcc tccttttcct cattg 35
<210> 17
   <211> 28
   <212> DNA
   <213> Bacillus subtilis
<220>
   <223> phoD-reverse-primer
<400> 17
   gggggatcca ttcaagatcc cctctctc 28
<210> 18
   <211> 31
   <212> DNA
   <213> Bacillus subtilis
<220>
   <223> pstS-forward-primer
<400> 18
   ggggaattct aaaaaaaacg ctcacatgat g 31
<210> 19
   <211> 31
   <212> DNA
   <213> Bacillus subtilis
<220>
   <223> pstS-mut-forward-primer
<400> 19
   ggggaattct aaaaaaaacg ctcacatgat g 31
<210> 20
   <211> 31
   <212> DNA
   <213> Bacillus subtilis
<220>
   <223> pstS-mut-reverse-primer
<400> 20
   cgggatccat ttcctaaatt cctcctttgt a 31
<210> 21
   <211> 29
   <212> DNA
   <213> Bacillus subtilis
<220>
   <223> pstS-reverse-primer
<400> 21
   cgggatccat ttcctaaatt cccccttcg 29